Europäisches Patentamt

European Patent Office

Office européen des brevets

(19)

(11) **EP 0 606 056 B1**

(12) **EUROPÄISCHE PATENTSCHRIFT**

(45) Veröffentlichungstag und Bekanntmachung des Hinweises auf die Patenterteilung:
**19.04.2000  Patentblatt 2000/16**

(51) Int. Cl.$^7$: **C07D 323/06**

(21) Anmeldenummer: **94100010.1**

(22) Anmeldetag: **03.01.1994**

(54) **Verfahren zur Herstellung von Trioxan**

Process for the preparation of trioxane

Procédé de préparation de trioxanne

(84) Benannte Vertragsstaaten:
**DE**

(30) Priorität: **06.01.1993 DE 4300138**

(43) Veröffentlichungstag der Anmeldung:
**13.07.1994  Patentblatt 1994/28**

(73) Patentinhaber: **Ticona GmbH**
**65451 Kelsterbach (DE)**

(72) Erfinder:
• **Emig, Gerhard, Prof. Dr.**
**D-91058 Erlangen (DE)**

• **Krüger, Benno, Dr.**
**D-65529 Waldems-Esch (DE)**
• **Kern, Frank, Dr.-Ing.**
**D-76870 Kandel (DE)**
• **Hoffmockel, Michael, Dr.**
**D-65527 Niedernhausen (DE)**
• **Mück, Karl-Friedrich, Dr.**
**D-65207 Wiesbaden (DE)**
• **Sextro, Günter, Dr.**
**D-65207 Wiesbaden (DE)**

(56) Entgegenhaltungen:
**DE-A- 3 106 476**

**Beschreibung**

Die Erfindung bezieht sich auf ein Verfahren zur kontinuierlichen Herstellung von Trioxan in der Gasphase durch heterogene Katalyse mit 11-Molybdo-1-vanado-phosphorsäure $H_4PVMO_{11}O_{40} * n H_2O$ (n = 0 - 32) (im folgenden 1-V-Säure) genannt) als Katalysator.

Trioxan kann aus wäßrigen Formaldehydlösungen mit sauren Katalysatoren hergestellt werden. Kennzeichnend für diese Prozesse ist der hohe Energieverbrauch zum Verdampfen von Wasser, das durch die Eduktströme in den Prozeß eingetragen wird.

Die DE-A-3106476 beschreibt ein Verfahren zur Herstellung von Trioxan durch Erhitzen und Umsetzen einer wäßrigen Formaldehydlösung in Gegenwart einer Heteropolysäure. Vorzugsweise werden Heteropolysäuren, die als Koordinationselement oder Koordinationselemente W, Mo, V oder Nb allein oder zu zweien oder mehreren enthalten, verwendet.

Es gibt verschiedene Vorschläge, Trioxan durch eine Gasphasentrimerisierung von Formaldehyd herzustellen, jedoch wird hierbei immer mit Formaldehydströmen von unterschiedlichem Wassergehalt gearbeitet. Durch das Arbeiten mit wasserhaltigem Formaldehyd treten durch Ablagerungen von Polyoxymethylen auf der Katalysatorenoberfläche Probleme auf.

Ein Verfahren zur Herstellung von Trioxan mittels eines sauren Harzionentauschers ist bekannt (DE-C-1 593 990). Ebenfalls bekannt ist ein Katalysator für die Gasphasentrimerisierung in Form von Phosphorsäure und Schwefelsäure auf $SiO_2$-Trägern (AT-B 252 913). Verschiedene Heteropolysäuren sind schon als Katalysatoren bei der Gasphasentrimerisierung von Formaldehyd eingesetzt worden (JP-A 59-25387). Jedoch sind die Ausbeuten nicht zufriedenstellend.

Es bestand die Aufgabe, die genannten Nachteile zu beseitigen.

Gelöst wurde die Aufgabe durch ein Verfahren, das sich von den bisherigen Verfahren durch den Katalysator, die Zusammensetzung des Eduktstroms und die hohe Selektivität des Katalysators, vor allem bei hohen Formaldehydpartialdrücken unterscheidet.

Die Erfindung beschreibt somit ein Verfahren zur Herstellung von Trioxan aus Formaldehyd in der Gasphase in Gegenwart eines Katalysators, bei dem als Katalysator 11-Molybdo-1-vanado-phosphorsäure $H_4PVMO_{11}O_{40} * n H_2O$ (n = 0 - 32) eingesetzt wird.

Als Träger für die Katalysatorsubstanz können inerte Materialien z. B. Pellets aus Siliciumcarbid, Siliciumdioxid oder Aluminiumoxid eingesetzt werden.

Als Presshilfen werden Stoffe verwendet, die sich bei der Reaktion inert verhalten, z. B. feinverteilte Kieselsäure ($SiO_2$), Aluminiumoxid oder -hydroxid.

Enthält der eingesetzte Gasstrom kein Wasser, so liegt der Katalysator unter Reaktionsbedingungen in wasserfreier Form vor.

Besonders hervorzuheben sind bei dem Verfahren folgende Vorteile

    1. Erreichen von hohen Raumzeitausbeuten (RZA) [kg/m$^3$*h]
    2. Keine Nebenprodukte
    3. Moderate Wärmeentwicklung im Synthesereaktor

Der Katalysator wird im allgemeinen in Substanz eingesetzt. Vorteilhaft ist aber die Verwendung von Träger oder Presshilfen.

Im allgemeinen verwendet man Formaldehyd mit einem Restwassergehalt von < 50 ppm, den man als wasserfreien Formaldehyd bezeichnet. Der für das Verfahren einsetzbare Formaldehyd kann aber auch einen geringen Wassergehalt aufweisen, d.h. der in den Reaktor eingespeiste Gasstrom kann bis zu 1 Gew.-% Wasser enthalten. Setzt man einen höheren Wassergehalt ein, so wird die RZA erheblich gesteigert, wobei der prozentuale Umsatz an Formaldehyd ebenfalls zunimmt.

Der Temperaturbereich für die Reaktion liegt bei 80°C bis 150°C. Bevorzugt ist der Bereich von 100°C bis 120°C.

Die Kontaktdauer, in der keine Nebenprodukte beim Verfahren auftreten, beträgt im allgemeinen 1 bis 30 Sekunden, vorzugsweise 1 bis 10 Sekunden. Längere Verweilzeiten sind möglich, jedoch ist dann die Gefahr der Bildung von Nebenprodukte gegeben.

Die Reaktion wird durch den Partialdruck von Formaldehyd beeinflußt. Der Katalysator hat über einen großen Druckbereich eine hohe Selektivität für die Bildung von Trioxan. Der Eingangspartialdruck von Formaldehyd beträgt im allgemeinen 0,5 bis 5 bar, vorzugsweise 0,5 bis 2 bar.

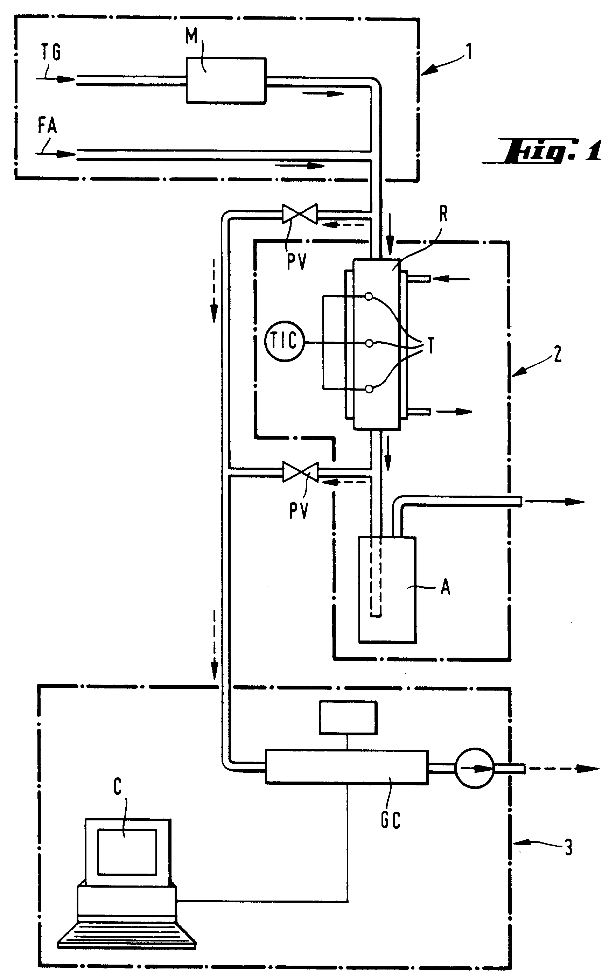

Die Apparatur zur Herstellung von Trioxan gemäß der Erfindung bestand aus drei Teilen (s. Fig. 1):

    1. Dosierung

2. Bildungsreaktor

3. Analytik

Zur Untersuchung wird Formaldehyd (FA) (= Eduktstrom) in die Apparatur eingeschleust und - wenn gewünscht - mit einem Trägergas vermischt. Als Trägergas können die Edelgase Helium, Argon, Krypton oder Xenon dienen, bevorzugt ist jedoch Stickstoff.

Der Bildungsreaktor (R) bestand - für die vorliegenden Versuche 1 bis 4 - aus einem Edelstahlrohrreaktor von 150 mm Länge und einem Durchmesser von 30 mm. Die Wärmezufuhr bzw. -abfuhr wurde durch einen Thermostaten (TIC) geregelt, als Wärmeüberträger wurde Silikonöl benutzt. Die Benutzung von Wärmeträgern wie Mineralölen ist ebenfalls möglich. An drei verschiedenen Stellen (T) des Reaktors wurde die Temperatur im Reaktor radial gemessen. Diese Temperaturen wurden während der Versuche aufgezeichnet und gaben Auskunft über den stabilen Betriebszustand des Reaktors während der Versuchsdauer.

Der aus dem Reaktor austretende Ausgangsstrom mit den entstandenen Reaktionsprodukten wurde in einer Absorptionsvorrichtung (A) in Wasser aufgefangen. Das gebildete Trioxan kann daraus in bekannter Weise durch Extraktion gewonnen werden.

Die quantitativen Untersuchungen zur Bildung von Trioxan in der Gasphase wurden mit einer Online-Analytik durchgeführt. Dabei wurden an zwei verschiedenen Stellen der Apparatur während des Betriebs Proben gezogen und in einem Gaschromatographen (GC) analysiert.

Die Anordnung der Apparatur für das Verfahren gemäß der Erfindung sowie die Dimensionen können natürlich den jeweiligen Bedingungen angepaßt werden.

Für die Versuche 5 und 6 wurde die Dimension des verwendeten Reaktors geändert. Hier betrug die Länge 130 mm und der Durchmesser 10 mm.

Versuchsbeschreibung:

Einzelne Parameter, die variiert wurden, werden bei den einzelnen Beispielen erläutert.

Bei den Versuchen 1 bis 4 wurde wasserfreier Formaldehyd eingesetzt.

Mit Hilfe eines Massendurchflußreglers (M) kann ein Trägergas (TG), vorzugsweise Stickstoff, dem Eduktstrom (FA) zugefügt werden. Es kann jedoch auch ohne Trägergas gearbeitet werden. Durch Einsatz eines Trägergases kann die Strömungsgeschwindigkeit des Eduktstromes variiert, z.B. erhöht, werden und damit die Verweilzeit im Reaktor (R) beeinflußt, z.B. erniedrigt werden. Im Reaktor (R) wurden verschiedene Mengen an Katalysator getestet. Vor und nach dem Reaktor (R) wurde die Gaszusammensetzung Online mit einem Gaschromatographen (GC) bestimmt. Aus den Zusammensetzungen der Gase wurde der Umsatz berechnet. Die Temperatur wurde über einen Thermostaten (TIC) geregelt und an drei verschiedenen Punkten (T) entlang der Katalysatorschüttung gemessen.

Die Probenahme zur Untersuchung der Eingangs- und Ausgangsströme wurde automatisch vorgenommen und erfolgte in regelmäßigen Abständen, z.B. alle sieben Minuten, wobei die Absperrvorrichtungen (PV) (pneumatische Kugelhähne) mit einem Gaschromatographen (GC) verbunden sind, der über einen Computer (C) gesteuert wird.

Die Angaben in den Tabellen der Beispiele werden wie folgt erklärt:

Der Molenbruch $X_{Form}$ wird auf die Gaszusammensetzung am Synthesereaktoreingang bezogen. Er wird nach Gleichung (1) berechnet.

$$X_{Form} = n^{o}_{Form}/n_{ges} = n^{o}_{Form}/(n^{o}_{Form} + n_{N2}) \qquad (1)$$

$n^{o}_{Form}$ : Formaldehydeingangsstrom [mol/h]
$n_{ges}$ : Gesamtstrom [mol/h]
$n_{N2}$ : Stickstoffstrom [mol/h]

Der experimentell ermittelte Umsatz $U_{exp}$ wird nach Gleichung (2) berechnet.

$$U_{exp} = (n^{o}_{Form} - n_{Form})/n^{o}_{Form} * 100 \qquad (2)$$

$n^{o}_{Form}$ : Formaldehydeingangsstrom [mol/h]
$n_{Form}$ : Formaldehydausgangsstrom [mol/h]

Der relative Umsatz $U_{rel}$ (Gleichung (3) ist das Verhältnis von experimentell ermitteltem Umsatz $U_{exp}$ und dem Gleichgewichtsumsatz $U_{glge}$, bestimmt aus der Gleichgewichtskonstanten nach Busfield und Merigold, ("The Gas-Phase Equilibrium between Trioxane and Formaldehyde", J. Chem. Soc (A), 1969 S. 2975).

$$U_{rel} = U_{exp}/U_{glge} *100 \qquad (3)$$

$U_{exp}$ : experimenteller Umsatz [%]

$U_{glge}$ : Gleichgewichtsumsatz [%]

Katalysatorherstellung:

Zur Herstellung der im allgemeinen zylindrischen Trägerkatalysatoren wird die 1-V-Säure, $H_4PVMo_{11}O_{40}$ * n $H_2O$ (n = 0 - 32), in Wasser gelöst, zum Tränken oder Imprägnieren der Träger verwendet. Dazu werden die Katalysatorträger mit der Lösung überschichtet, die in den Poren der Träger enthaltene Luft durch Verminderung des Drucks im Imprägniergefäß abgesaugt, die überstehende Lösung abgegossen und die imprägnierten Träger bei 373 K an der Luft getrocknet. Sollen die Träger nur getränkt werden, kann die Evakuierung entfallen.

Trägerlose Katalysatoren werden durch Vermischen von gesiebter 1-V-Säure, Korngröße 150 µm mit der gewünschten Menge an Presshilfe und anschließendes Verpressen zu Pellets der gewünschten Größe, z. B. in zylindrischer Form, hergestellt.

Für die Herstellung des Katalysators gemäß Beispiel 2 wurden z. B. 3,8 g 1-V-Säure in 60 $cm^3$ dest. Wasser gelöst und der Träger aus Siliciumcarbid wie oben beschrieben behandelt. Man erhält auf diese Weise Pellets, die bei einer Katalysatorschüttung von 50 $cm^3$ mit einer Menge von 1,69 g 1-V-Säure beaufschlagt sind.

Beispiele:

1) 61,25 g des Katalysators 1-V-Säure mit 30 % $SiO_2$ als Presshilfe wurden in Form von zylindrischen Pellets (Durchmesser 5 mm, Höhe 5 mm) im Reaktor R eingesetzt. Das Volumen der Katalysatorschüttung betrug 56,2 $cm^3$. Folgende Umsätze konnten bei einer Selektivität von 1 erreicht werden, d. h. es wurden keine Nebenprodukte erhalten. Der Gesamtdruck betrug 1,050 bar.

| Temp. [K] | Verweil-zeit [s] | $n^o_{Form}$ [mol/h] | Molen-bruch | $U_{exp}$ [%] | $U_{rel}$ [%] | RZA [kg/$m^3$h] |
|---|---|---|---|---|---|---|
| 358 | 3,4 | 0,692 | 0,267 | 32,03 | 100 | 118,5 |
| 363 | 4,6 | 0,962 | 0,492 | 35,2 | 87,5 | 180,96 |
| 368 | 3,6 | 1,54 | 0,611 | 40,2 | 88,9 | 329,89 |

## Tab 1. Umsätze bei verschiedenen Temperaturen

2) 1,69 g des Katalysators 1-V-Säure auf 46,15 g zylindrischer SiC-Pellets (Durchmesser 6 mm, Höhe 6 mm) wurden im Reaktor R eingesetzt. Folgende Umsätze konnten bei einer Selektivität von 1 erreicht werden. Der Eingangspartialdruck von Formaldehyd betrug 1075 mbar, das Volumen der Katalysatorschüttung betrug 50 $cm^3$.

| Temp. [K] | Verweil-zeit [s] | $n^{o}_{Form}$ [mol/h] | Molen-bruch | $U_{exp}$ [%] | $U_{rel}$ [%] | RZA [kg/m³h] |
|---|---|---|---|---|---|---|
| 383 | 11,25 | 0,516 | 0,6 | 17,1 | 50,6 | 53,0 |
| 380,5 | 11,25 | 0,516 | 0,6 | 29,2 | 76,8 | 90,5 |
| 378 | 11,25 | 0,517 | 0,6 | 33,76 | 79,4 | 104,8 |

Tab 2. Umsätze bei verschiedenen Temperaturen

3) 0,563 g des Katalysators 1-V-Säure auf 16,7 g zylindrischer SiC-Pellets (Durchmesser 6 mm, Höhe 6 mm) wurden im Reaktor R eingesetzt. Folgende Umsätze konnten bei einer Selektivität von 1 erreicht werden. Der Eingangspartialdruck von Formaldehyd betrug 1150 mbar, das Volumen der Katalysatorschüttung betrug 16,6 cm³.

| Temp. [K] | Verweil-zeit [s] | $n^{o}_{Form}$ [mol/h] | Molen-bruch | $U_{exp}$ [%] | $U_{rel}$ [%] | RZA [kg/m³h] |
|---|---|---|---|---|---|---|
| 383 | 5,1 | 0,386 | 0,6 | 29,6 | 81,3 | 206,8 |
| 380,5 | 5,1 | 0,378 | 0,6 | 35,2 | 82,4 | 240,8 |

Tab 3. Umsätze bei verschiedenen Temperaturen

4) 0,9 g des Katalysators 1-V-Säure auf 26,65 g zylindrischer SiC-Pellets (Durchmesser 6 mm, Höhe 6 mm) wurden im Reaktor R eingesetzt. Folgende Umsätze konnten bei einer Selektivität von 1 erreicht werden. Der Eingangspartialdruck von Formaldehyd betrug 950 mbar, das Volumen der Katalysatorschüttung betrug 26,7 cm³.

| Temp. [K] | Verweil-zeit [s] | $n^o_{Form}$ [mol/h] | Molen-bruch | $U_{exp}$ [%] | $U_{rel}$ [%] | RZA [kg/m$^3$h] |
|---|---|---|---|---|---|---|
| 383 | 5,1 | 0,375 | 0,6 | 13,67 | 45,5 | 57,6 |
| 380,5 | 5,1 | 0,374 | 0,6 | 17,2 | 48,8 | 72,4 |
| 378 | 5,1 | 0,375 | 0,6 | 21,9 | 56,6 | 92,3 |
| 375,5 | 5,1 | 0,374 | 0,6 | 22,51 | 52,1 | 94,8 |

## Tab 4. Umsätze bei verschiedenen Temperaturen

Die Beispiele 1 bis 4 zeigen, daß der Katalysator eine hohe Selektivität für die Bildung von Trioxan besitzt. Die Aktivität des Katalysators steigt mit wachsendem Formaldehydpartialdruck und sinkender Reaktortemperatur. Es können sowohl hohe Umsätze nahe dem Gleichgewichtsumsatz als auch hohe Raumzeitausbeuten erreicht werden. In allen aufgeführten Beispielen wurde Stickstoff als Trägergas und Referenzgas für die Analysen eingesetzt. Der Wassergehalt des Formaldehydeingangsstromes lag bei den Beispielen 1 bis 4 geringer als 50 ppm. Die verwendeten Katalysatoren zeigten nach einer jeweiligen Versuchsdauer von 300 Stunden keinerlei Anzeichen einer Desaktivierung. Durch die Verwendung von wasserfreiem Formaldehyd kann mit höheren Formaldehydeingangspartialdrücken gearbeitet werden als mit wasserhaltigem Formaldehyd. Der Zersetzungsdruck von Paraformaldehyd mit einem Wassergehalt von 5 % liegt zum Beispiel bei einer Temperatur von 380,5 K bei 0,97 bar. In Beispiel 3 konnte bei Verwendung von wasserfreiem Formaldehyd der Formaldehydeingangspartialdruck auf 1,15 bar bei dieser Temperatur gesteigert werden, ohne daß eine Kondensation von Formaldehyd im Reaktor eintrat. Mit der Steigerung des Eingangspartialdruckes ist eine deutliche Steigerung des maximal möglichen Gleichgewichtsumsatzes von 35 % auf 40 % und damit der Wirtschaftlichkeit des Prozesses verbunden.

In den folgenden Beispielen 5 und 6 wird der positive Einfluß eines bestimmten Wassergehaltes in dem Reaktionsgas beschrieben (Beispiel 5) im Vergleich zu einem wasserfreien Ansatz (Beispiel 6). Der Reaktor gemäß Fig. 1 wies eine Länge von 130 mm und einen Durchmesser von 10 mm auf. Der Wassergehalt betrug 1 Gew.-%, wobei der Stickstoffstrom (TG) mit dieser Menge beladen wurde.

In zwei aufeinanderfolgenden Versuchen wurde ein Eingangsstrom unter den in Tab. 5 angeführten Bedingungen über die Katalysatorschüttung geleitet.

Tabelle 5

| | | Beispiel 5 | Beispiel 6 (Vergleich) |
|---|---|---|---|
| Gesamtdruck | [mbar] | 1120 | 1120 |
| Partialdruck $N_2$ | [mbar] | 183 | 200 |
| Partialdruck $H_2O$ | [mbar] | 17 | 0 |
| Partialdruck $CH_2O$ | [mbar] | 920 | 920 |
| Reaktortemperatur | [°C] | 110 | 110 |
| Katalysator $H_4PVMO_{11}O_{40}$ | [mg] | 85 auf 10 cm$^3$ zylindrische SiC-Pellets, 1 x 1 mm | 85 auf 10 cm$^3$ zylindrische SiC-Pellets, 1 x 1 mm |

Tabelle 5 (fortgesetzt)

| | | Beispiel 5 | Beispiel 6 (Vergleich) |
|---|---|---|---|
| Reaktionsvolumen | [cm$^3$] | 10 | 10 |
| n$^\circ_{Form}$ | [mol/l] | 0,22 | 0,22 |
| U$_{exp}$ (CH$_2$O) | [%] | 12 | 7,5 |
| RZA | [kg/m$^3 \cdot$ h] | 84 | 50 |

Das Produktgemisch am Reaktorausgang bestand aus Formaldehyd, Wasser, Stickstoff und Trioxan, wobei Trioxan mit einer Selektivität von 1 erhalten wurde.

Die Eingangs- und Ausgangsströme wurden durch Gaschromatographie untersucht (Fig. 1). Durch den Wassergehalt im Eingangsstrom wird die Aktivität des Katalysators erheblich gesteigert.

**Patentansprüche**

1. Verfahren zur Herstellung von Trioxan aus Formaldehyd in der Gasphase in Gegenwart eines Katalysators, dadurch gekennzeichnet, daß als Katalysator 11-Molybdo-1-vanado-phosphorsäure $H_4PVMo_{11}O_{40}$ * n $H_2O$ (n = 0 - 32) eingesetzt wird.

2. Verfahren nach Anspruch 1, dadurch gekennzeichnet, daß der Katalysator auf inerte Trägermaterialien aufgebracht wird oder mit inerten Presshilfen verpreßt wird.

3. Verfahren nach Anspruch 1 oder 2, dadurch gekennzeichnet, daß als Trägermaterial Siliciumcarbid und als Presshilfe feinverteilte Kieselsäure eingesetzt wird.

4. Verfahren nach einem oder mehreren der Ansprüche 1 bis 3, dadurch gekennzeichnet, daß wasserfreier Formaldehyd eingesetzt wird.

5. Verfahren nach einem oder mehreren der Ansprüche 1 bis 3, dadurch gekennzeichnet, daß Formaldehyd mit einem geringen Wassergehalt von bis zu 1 Gew.-% eingesetzt wird.

6. Verfahren nach einem oder mehreren der Ansprüche 1 bis 5, dadurch gekennzeichnet, daß die Reaktion bei 80 bis 150 °C, vorzugsweise bei 100 bis 120 °C durchgeführt wird.

7. Verfahren nach einem oder mehreren der Ansprüche 1 bis 6, dadurch gekennzeichnet, daß die Kontaktdauer 1 bis 30, vorzugsweise 1 bis 10 Sekunden beträgt.

8. Verfahren nach einem oder mehreren der Ansprüche 1 bis 7, dadurch gekennzeichnet, daß der Eingangspartialdruck des Formaldehyds 0,5 bis 5 bar, vorzugsweise 0,5 bis 2 bar beträgt.

9. Verfahren nach einem oder mehreren der Ansprüche 1 bis 8, dadurch gekennzeichnet, daß die Reaktion in Anwesenheit oder Abwesenheit eines Trägergases durchgeführt wird.

10. Verfahren nach Anspruch 9, dadurch gekennzeichnet, daß Stickstoff als Trägergas verwendet wird.

**Claims**

1. A process for the preparation of trioxane from formaldehyde in the gas phase in the presence of a catalyst, wherein the catalyst employed is 11-molybdo-1-vanadophosphoric acid, $H_4PVMo_{11}O_{40}$ • n $H_2O$ (n = 0-32).

2. The process as claimed in claim 1, wherein the catalyst is applied to an inert support material or pressed with inert pressing aids.

3. The process as claimed in claim 1 or 2, wherein the support material employed is silicon carbide and the pressing aid employed is finely divided silica.

4. The process as claimed in one or more of claims 1 to 3, wherein anhydrous formaldehyde is employed.

5. The process as claimed in one or more of claims 1 to 3, wherein the formaldehyde employed has a low water content of up to 1% by weight.

6. The process as claimed in one or more of claims 1 to 5, wherein the reaction is carried out at from 80 to 150°C, preferably at from 100 to 120°C.

7. The process as claimed in one or more of claims 1 to 6, wherein the contact duration is from 1 to 30 seconds, preferably from 1 to 10 seconds.

8. The process as claimed in one or more of claims 1 to 7, wherein the partial pressure of the formaldehyde at the inlet is from 0.5 to 5 bar, preferably from 0.5 to 2 bar.

9. The process as claimed in one or more of claims 1 to 8, wherein the reaction is carried out in the presence or absence of a carrier gas.

10. The process as claimed in claim 9, wherein the carrier gas used is nitrogen.

**Revendications**

1. Procédé de préparation de trioxane à partir de formaldéhyde en phase gazeuse en présence d'un catalyseur, caractérisé en ce que l'on utilise de l'acide 11-molybdo-1-vanadophosphorique $H_4PVMo_{11}O_{40}*nH_2O$ (n = 0-32) en tant que catalyseur.

2. Procédé selon la revendication 1, caractérisé en ce que le catalyseur est appliqué sur des matériaux de support inertes ou comprimé à l'aide d'auxiliaires de pressage inertes.

3. Procédé selon la revendication 1 ou 2, caractérisé en ce que l'on utilise du carbure de silicium en tant que matériau de support et de l'acide silicique finement divisé en tant qu'auxiliaire de pressage.

4. Procédé selon l'une ou plusieurs des revendications 1 à 3, caractérisé en ce que l'on utilise du formaldéhyde anhydre.

5. Procédé selon l'une ou plusieurs des revendications 1 à 3, caractérisé en ce que l'on utilise du formaldéhyde ayant une faible teneur en eau allant jusqu'à 1% en poids.

6. Procédé selon l'une ou plusieurs des revendications 1 à 5, caractérisé en ce que la réaction est réalisée entre 80 et 150°C, de préférence entre 100 et 120°C.

7. Procédé selon l'une ou plusieurs des revendications 1 à 6, caractérisé en ce que la période de contact est de 1 à 30, de préférence de 1 à 10 secondes.

8. Procédé selon l'une ou plusieurs des revendications 1 à 7, caractérisé en ce que la pression partielle de départ du formaldéhyde est de 0,5 à 5 bars, de préférence de 0,5 à 2 bars.

9. Procédé selon l'une ou plusieurs des revendications 1 à 8, caractérisé en ce que la réaction est réalisée en présence ou en l'absence d'un gaz vecteur.

10. Procédé selon la revendications 9, caractérisé en ce que l'on utilise de l'azote en tant que gaz vecteur.

_Fig. 1_